# EUROPEAN PATENT SPECIFICATION

(11) **EP 3 662 816 B1**
(45) Date of publication and mention of the grant of the patent: **14.08.2024**
(21) Application number: 18842119.2
(22) Date of filing: 31.07.2018
(51) Int. Cl.: A61B 5/00, A61B 5/251, A61D 7/00, A61D 3/00, A61B 5/291, A61B 5/296, A61B 5/28

(54) **BIOMETRIC SIGNAL-MEASURING APPARATUS EQUIPPED WITH TRAP UNIT FOR MEASURING BIOMETRIC SIGNAL OF MICRO-ANIMAL**
VORRICHTUNG ZUR MESSUNG BIOMETRISCHER SIGNALE MIT FANGEINHEIT ZUR MESSUNG DES BIOMETRISCHEN SIGNALS EINES MIKROTIERS
APPAREIL DE MESURE DE SIGNAUX BIOMÉTRIQUES MUNI D'UNE UNITÉ DE PIÈGE POUR MESURER DES SIGNAUX BIOMÉTRIQUES DE MICRO-ANIMAUX

(30) Priority: 31.07.2017 KR 20170097217
(43) Date of publication of application: 10.06.2020
(73) Proprietor: Daegu Gyeongbuk Institute of Science and Technology, Daegu 42988 (KR)
(72) Inventor: KIM, So Hee, Daegu 42001 (KR); CHO, Sung Joon, Gwangju 62220 (KR); KANG, Yang Jun, Gwangju 61452 (KR)
(74) Representative: Zimmermann, Tankred Klaus
(86) International application number: PCT/KR2018/008654
(87) International publication number: WO 2019/027216

(56) References cited:
- WO-A1-03/096002
- WO-A1-2012/154552
- WO-A1-2017/027838
- JP-A- 2016 021 947
- KR-B1- 101 617 799
- KR-B1- 101 693 493
- KR-B1- 101 693 493
- US-A1- 2016 178 604
- BISCHEL LAUREN L. ET AL: "Zebrafish Entrapment By Restriction Array (ZEBRA) device: a low-cost, agarose-free zebrafish mounting technique for automated imaging", LAB ON A CHIP, vol. 13, no. 9, 4 March 2013 (2013-03-04), pages 1732, XP055803176, ISSN: 1473-0197, DOI: 10.1039/c3lc50099c
- HONG SOONGWEON ET AL: "A Novel Long-term, Multi-Channel and Non-invasive Electrophysiology Platform for Zebrafish", SCIENTIFIC REPORTS, vol. 6, no. 1, 16 June 2016 (2016-06-16), XP055803177, DOI: 10.1038/srep28248
- CHAUDHARI GIRISH HARI ET AL: "Optimization of the adult zebrafish ECG method for assessment of drug-induced QTc prolongation", JOURNAL OF PHARMACOLOGICAL AND TOXICOLOGICAL METHODS, ELSEVIER, NEW YORK, NY, US, vol. 67, no. 2, 24 January 2013 (2013-01-24), pages 115 - 120, XP028994553, ISSN: 1056-8719, DOI: 10.1016/J.VASCN.2013.01.007
- ZHU FENG ET AL: "Fishing on chips: Up-and-coming technological advances in analysis of zebrafish and X enopus embryos : Fishing on Chips", NIH PUBLIC ACCESS AUTHOR MANUSCRIPT, vol. 85, no. 11, 6 October 2014 (2014-10-06), pages 921 - 932, XP055803172, ISSN: 1552-4922, DOI: 10.1002/cyto.a.22571

## Description

### [Technical Field]

The present invention relates to a biological-signal measuring apparatus equipped with a trap unit for measuring biological signals of micro-vertebrates and more specifically, to a biological-signal measuring apparatus equipped with a trap unit for measuring biological signals of micro-vertebrates by which simultaneous measurement of biological signals of a plurality of micro-vertebrates can be rapidly performed while lives of the micro-vertebrates are maintained.

### [Background Art]

In general, electromyography (EMG) is a testing technique for evaluating a function of a muscle by recording an action potential occurring during contraction of a muscular fiber, in which a muscle reaction to neurostimulation is tested by detecting an electrical change in the muscle. A muscle is controlled by a nerve, and a fine current continuously flows in the muscle itself. Therefore, when the fine current is checked by a needle, an electrode, or the like and is recorded by using an electromyograph, it is possible to know whether the muscle works appropriately from a peripheral nerve to the muscle itself. Hence, the electromyography is used mainly when a problem arises in the peripheral nerve and the muscle and, particularly, is used to find a state of a muscle such as muscle atrophy or neurological disorder which results in muscle weakness.

Recently, there is developed a technology for performing tests of electromyography, electroencephalography, electrocardiography, and the like on micro-vertebrates having genetic information similar to human genetic information. As an example, there is developed a system for performing electromyography on a zebrafish among micro-vertebrates, the zebrafish being a vertebrate having genetic information similar to human genetic information.

However, in an electromyography system for micro-vertebrates in the related art, it is difficult to perform simultaneous measurement of micro-vertebrates, and it is inconvenient to insert the micro-vertebrates into agarose gel or a coil electrode. In addition, the electromyography system for micro-vertebrates in the related art has a problem in that it is complicated to perform drug screening.

### [Citation List]

JP 2002-085362 A,
US 2016/178604 A1,
BISCHEL LAUREN L. ET AL, "Zebrafish Entrapment By Restriction Array (ZEBRA) device: a low-cost, agarose-free zebrafish mounting technique for automated imaging", LAB ON A CHIP, (20130304), vol. 13, no. 9, doi:10.1039/c3lc50099c, ISSN 1473-0197, page 1732,
HONG SOONGWEON ET AL, "A Novel Long-term, MultiChannel and Non-invasive Electrophysiology Platform for Zebrafish", SCIENTIFIC REPORTS, (20160616), vol. 6, no. 1, doi:10.1038/srep28248,
WO 2017027838 A1,
CHAUDHARI GIRISH HARI ET AL, "Optimization of the adult zebrafish ECG method for assessment of drug-induced QTc prolongation", JOURNAL OF PHARMACOLOGICAL AND TOXICOLOGICAL METHODS, ELSEVIER, NEW YORK, NY, US, (20130124), vol. 67, no. 2, doi:10.1016/J.VASCN.2013.01.007, ISSN 1056-8719, pages 115 - 120

### [Summary of Invention]

In order to solve such problems described above, an object of the present invention is to provide a biological-signal measuring apparatus equipped with a trap unit for measuring biological signals of micro-vertebrates by which simultaneous measurement of biological signals of a plurality of micro-vertebrates can be rapidly performed while lives of the micro-vertebrates are maintained.

This object is achieved by a biological-signal measuring apparatus according to claim 1.

In an embodiment of the present invention, the trap unit may further have comparison electrodes provided to be in contact with the micro-vertebrates when each of the micro-vertebrates is fixed to each of the trap channels. When the electrode portion is inserted into the micro-vertebrates, a potential difference between the electrode portion and the comparison electrode may be measured to measure biological signals of the micro-vertebrates.

In an embodiment of the present invention, the biological-signal measuring apparatus may further include a stage unit that is provided below the trap unit and controls a position of the trap unit; and a base unit to which, on an upper side, the stage unit and the lifting/lowering unit are coupled.

In an embodiment of the present invention, the stage unit may have an insertion body having inside a groove into which the trap unit is inserted and fixed; a fixing body which is provided on an outer surface of the insertion body and fixes the insertion body; a sliding section which is provided below the insertion body and is provided to be slidable in an X direction and a Y direction; a main stage body which is provided below the sliding section; a width adjuster which is connected to the main stage body and moves the sliding section in the X direction; and a length adjuster which is connected to the main stage body and moves the sliding section in the Y direction.

In an embodiment of the present invention, the base unit may have a base section which is coupled to the lifting/lowering unit and the stage unit at a top part of the base section and forms a body; and a rail section on which the lifting/lowering unit is slidable in a length direction of the base section.

In an embodiment of the present invention, the lifting/lowering unit may further have a vertical frame which slides along the rail section in the length direction of the base section; a horizontal frame provided to be extended from the vertical frame in one direction so as to be liftable and lowerable in a length direction of the vertical frame; and the electrode portion which is coupled to the horizontal frame and is extended downward. The electrode portion may include working electrodes corresponding to the trap channels in number.

According to a configuration of the present invention, in order to achieve the object described above, there is provided an electromyography system and the biological-signal measuring apparatus equipped with a trap unit for measuring biological signals of micro-vertebrates.

According to a configuration of the present invention, in order to achieve the object described above, there is provided an electrocardiography system and the biological-signal measuring apparatus equipped with a trap unit for measuring biological signals of micro-vertebrates.

According to a configuration of the present invention, in order to achieve the object described above, there is provided an electroencephalography or a brainwave test system and the biological-signal measuring apparatus equipped with a trap unit for measuring biological signals of micro-vertebrates.

### [Advantageous Effects of Invention]

According to the configuration described above, effects of the present invention are as follows. It is possible to easily fix multiple micro-vertebrates, and thus it is possible to simultaneously measure biological signals of the multiple micro-vertebrates.

In addition, according to the present invention, since a fluid containing a drug is injected together with the micro-vertebrates via an injection chamber, it is easy to screen the drug and to easily change a type of drug.

In addition, according to the present invention, the fluid is continuously supplied via the injection chamber, and thus it is possible to maintain lives of the micro-vertebrates while biological signals of the micro-vertebrates are measured.

Effects of the present invention are construed not to be limited to the above-mentioned effects but to include every effect that can be derived from configurations of the invention described in the detailed description of the embodiments and claims of the present invention.

### [Brief Description of Drawings]

FIG. 1 is a perspective view of a biological-signal measuring apparatus equipped with a trap unit for measuring biological signals of micro-vertebrates according to an embodiment of the present invention.
FIG. 2 is a top view of the trap unit according to the embodiment of the present invention.
FIG. 3 is a view illustrating a top surface of the trap unit according to the embodiment of the present invention.
FIG. 4 is a view illustrating a top surface of a trap unit according to an example not covered by the claims.
FIG. 5 is a view illustrating a state of measuring biological signals of micro-vertebrates fixed to the trap unit according to the embodiment of the present invention.
FIG. 6 is a graph showing a result of electromyogram measurement of micro-vertebrates by using the biological-signal measuring apparatus equipped with a trap unit for measuring biological signals of micro-vertebrates according to the embodiment of the present invention.
FIG. 7 is a flowchart of a method for measuring biological signals of micro-vertebrates by using the biological-signal measuring apparatus equipped with a trap unit for measuring biological signals of micro-vertebrates, the method not falling within the scope of the claims.
FIG. 8 is a picture showing a state of measuring biological signals of micro-vertebrates fixed to the trap unit according to the embodiment of the present invention.

### [Description of Preferred Embodiment]

According to a preferred embodiment of the present invention, there is provided a biological-signal measuring apparatus equipped with a trap unit for measuring biological signals of micro-vertebrates, the biological-signal measuring apparatus including: a trap unit having multiple trap channels to which multiple micro-vertebrates are fixed; and a lifting/lowering unit that inserts an electrode portion into the micro-vertebrates fixed to the trap unit and measures biological signals. The trap channels have a shape formed to fix the head part of the micro-vertebrates put into the trap unit.

### [Description of Embodiments]

Hereinafter, the present invention will be described with reference to the accompanying drawings. However, the present invention can be realized as various different examples and, thus, is not limited to embodiments described here. Besides, a part irrelevant to the description is omitted from the drawings in order to clearly describe the present invention, and similar reference signs are assigned to similar parts through the entire specification.

In the entire specification, a case where a certain part "is connected to (accesses, is in contact with, or is coupled to)" another part includes not only a case where the parts are "directly connected" to each other, but also a case where the parts are "indirectly connected" to each other with another member interposed therebetween. In addition, when a certain part "includes" a certain configurational element, this means that another configurational element is not excluded but the configurational element can be further included, unless specifically described otherwise.

Terms used in this specification are only used to describe a specific embodiment and are not intentionally used to limit the present invention thereto. A singular form includes a plural form, unless obviously implied otherwise in context. In this specification, words such as "to include" or "to have" is construed to specify that a feature, a number, a step, an operation, a configurational element, a member, or an assembly thereof described in the specification is present and not to exclude presence or a possibility of addition of one or more other features, numbers, steps, operations, configurational elements, members, or assemblies thereof in advance.

Hereinafter, embodiments of the present invention will be described in detail with reference to the accompanying drawings.

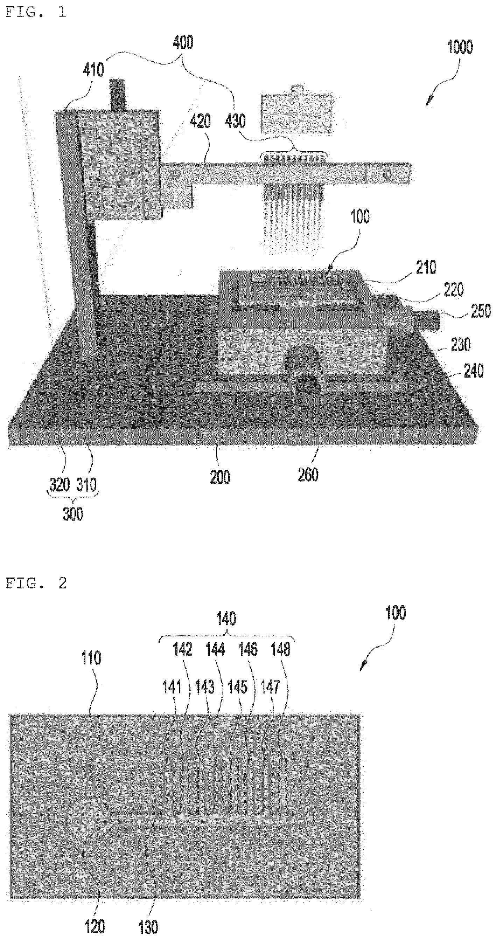

FIG. 1 is a perspective view of a biological-signal measuring apparatus equipped with a trap unit for measuring biological signals of micro-vertebrates according to an embodiment of the present invention. FIG. 2 is a top view of the trap unit according to the embodiment of the present invention.

As illustrated in FIGS. 1 and 2, a biological-signal measuring apparatus 1000 equipped with a trap unit for measuring biological signals of micro-vertebrates includes a trap unit 100, a stage unit 200, a base unit 300, and a lifting/lowering unit 400.

The trap unit 100 fixes micro-vertebrates and includes a substrate 110, an injection chamber 120, a transfer channel 130, multiple trap channels 140, a comparison electrode 150, and a discharge section 160.

The substrate 110 can form a main body of the trap unit 100 and can be provided to be coupled and fixed to the stage unit 200.

The injection chamber 120 can be provided in the substrate 110, and the micro-vertebrates and a fluid can be injected into the injection chamber. Here, the micro-vertebrates are living organisms having genetic information similar to human genetic information, and the micro-vertebrates are zebrafish.

In addition, a fluid injected into the injection chamber 120 can contain a drug and water. The micro-vertebrates are injected together with water and a drug into the injection chamber 120, and thus a life of the micro-vertebrates can be maintained during measuring biological signals such that it is possible to improve reliability of an experiment. Besides, it is easy to change a drug which is injected into the injection chamber 120, and thus it is easy to measure the biological signals of the micro-vertebrates when each drug is injected.

The transfer channel 130 can be provided to be extended from the injection chamber 120 toward one side and can form a flow channel to transfer the micro-vertebrates and the fluid injected into the injection chamber 120 to the trap channels 140.

The trap channels 140 can be provided to be extended from the transfer channel 130 in one direction, and one or more trap channels can be provided. That is, eight trap channels 140 are illustrated in the drawing; however, the number of trap channels is not limited thereto.

In addition, the trap channels 140 can have a shape formed to fix the head part h of the micro-vertebrates put into the trap unit 100.

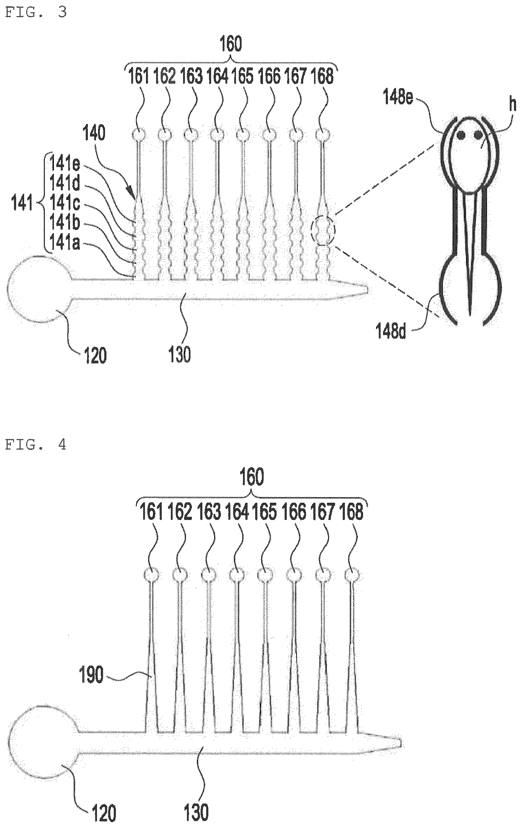

FIG. 3 is a view illustrating a top surface of the trap unit according to the embodiment of the present invention.

As illustrated in FIG. 3, a plurality of traps 141a, 142b, 141c, 141d, and 141e are formed in the trap channels 140 in a length direction thereof, and the traps have a sectional area which decreases as the trap is positioned downstream.

Specifically, the trap 141b positioned downstream has a smaller sectional area than the trap 141a positioned relatively upstream. In the trap channels 140 provided as described above, the head part h of the micro-vertebrates can be caught on and fixed to a trap having a size corresponding to that of the head part h while micro-vertebrates which are injected into the injection chamber 120 and have different sizes pass through the trap channels 140.

As an example, the micro-vertebrates having the head part h smaller than the trap 141a, which is positioned relatively upstream and has a large sectional area, can pass through the trap 141a; however, the head part h is larger than the trap 141e positioned relatively downstream, does not pass therethrough, and can be caught on and fixed to the trap.

FIG. 4 is a view illustrating a top surface of a trap unit according to an example not covered by the claims.

As illustrated in FIG. 4, the trap unit can be characterized by having multiple trap channels 190 having an area which gradually decreases toward downstream.

Specifically, the trap channels 190 can be provided to have a shape with a sectional area that decreases at a constant rate toward downstream, and thus the trap channels 190 can be provided to cause the head part h to be caught on and fixed to a portion having a sectional area corresponding to the head part h of the micro-vertebrates while the micro-vertebrates pass through the trap channels.

The trap channels 140 according to the embodiment provided as described above and the trap channels 190 according to the example are provided to have a configuration in which the micro-vertebrates injected from the injection chamber 120 are fixed thereto while moving toward the discharge section 160 without any control.

In the related art, in order to measure biological signals of micro-vertebrates, the micro-vertebrates need to be inserted into agarose gel or a fixing device such as a coil electrode, and thus it is difficult to perform fixing in a state where lives of the micro-vertebrates are maintained. In addition, zebrafish have a size of about 3 mm, and thus time and effort are required to fix micro-vertebrates individually by using a separate device.

However, the trap channels of the present invention are convenient in that several micro-vertebrates can be simultaneously and rapidly fixed, and the micro-vertebrates can be fixed without any control while lives of the micro-vertebrates are maintained.

In addition, the micro-vertebrates can be directly positioned in the trap channels 140 according to the embodiment or in the trap channels 190 according to the example.

The comparison electrode 150 can be provided to be in contact with the micro-vertebrates when the micro-vertebrates are fixed to the trap channels 140. Regarding the comparison electrode 150, when an electrode portion 430 of the lifting/lowering unit 400 is inserted into the micro-vertebrates, a potential difference between the electrode portion 430 and the comparison electrode 150 can be measured to measure biological signals of the micro-vertebrates.

The discharge section 160 is provided to be extended downstream of the trap chamber 140, and thus micro-vertebrates and a fluid entering the injection chamber 120 can be discharged through the discharge section 160. At this point, the micro-vertebrates fixed to the trap channels 140 can be discharged by increasing pressure of the fluid injected into the injection chamber 120 or can be discharged by injecting a solution into the injection chamber 120.

The stage unit 200 can be provided below the trap unit 100 and can control a position of the trap unit 100. Besides, the stage unit 200 includes an insertion body 210, a fixing body 220, a sliding section 230, a main stage body 240, a width adjuster 250, and a length adjuster 260.

The insertion body 210 can be provided below the trap unit 100 and can have a groove inside into which the trap unit 100 can be inserted and fixed. Specifically, a groove can be formed on an inner side of a top surface of the insertion body 210, and the substrate 110 of the trap unit 100 can be seated in the groove. The insertion body 210 can be fixed in a state where the substrate 110 is seated into the groove.

The fixing body 220 can be provided on an outer surface of the insertion body 210 and can be provided to fix the insertion body 210. Specifically, the fixing body 220 can be provided on a corner of an outer surface of the insertion body 210 to fix the insertion body 210; however, an example, in which the insertion body 210 is fixed, is not necessarily limited to the embodiment. That is, examples of the fixing body 220 include every example in which the insertion body 210 can be fixed.

The sliding section 230 can be provided below the insertion body 210 and can be provided to be slidable in an X direction and a Y direction. Specifically, the sliding section 230 can be provided to have an upper part to which the insertion body 210 is fixed, and the sliding section can be coupled to an upper part of the main stage body 240 to be described below and can be coupled to be slidable in the X direction and the Y direction.

The main stage body 240 can be provided below the sliding section 230 and can be provided to be fixed to an upper part of the base unit 300.

The width adjuster 250 can be connected to the main stage body 240 and can move the sliding section 230 in the X direction. Here, the X direction can be referred to as a width direction of the main stage body 240.

The length adjuster 260 can be connected to the main stage body 240 and can move the sliding section 230 in the Y direction. Here, the Y direction can be referred to as a length direction of the main stage body 240.

The width adjuster 250 and the length adjuster 260 provided as described above can move the sliding section 230 in the X direction and the Y direction so as to adjust an inserting site of the electrode portion 430 into the micro-vertebrates fixed to the trap channels 140.

The stage unit 200 and the lifting/lowering unit 400 can be coupled to the base unit 300 on an upper side thereof, and the base unit includes a base section 310 and a rail section 320.

The base section 310 is coupled to the stage unit 200 and the lifting/lowering unit 400 at a top part of the base section and forms a body of the base unit 300. Here, the base section 310 is provided to have a quadrangular plate shape; however, the shape of the base section 310 is not limited to the illustrated shape.

The rail section 320 can be formed at a top surface of the base section 310 to be extended in a length direction of the base section 310 and can be provided to allow the lifting/lowering unit 400 to be seated and slide on the rail section 320. That is, the rail section 320 can be provided to allow the lifting/lowering unit 400 to slide in the length direction of the base section 310.

The lifting/lowering unit 400 can insert the electrode portions 430 into the micro-vertebrates fixed to the trap unit 100 and can measure biological signals thereof, and the lifting/lowering unit includes a vertical frame 410, a horizontal frame 420, and the electrode portions 430.

That is, the vertical frame 410 can be provided to slide along the rail section 320 in the length direction of the base section 310. Specifically, the vertical frame 410 can be provided to be vertically extended upward from the base section 310, and a lower end of the vertical frame can be seated on the rail section 320 to slide in a length direction of the rail section 320.

The horizontal frame 420 can be provided to be extended from the vertical frame 410 in one direction so as to be liftable and lowerable in a length direction of the vertical frame 410. Specifically, the horizontal frame 420 can be provided to be extended from the vertical frame 410 in a direction toward the trap unit 100, and one end of the horizontal frame can be coupled to the vertical frame 410 to be liftable upward and lowerable downward.

The electrode portions 430 can be coupled to the horizontal frame 420 to be extended downward. Besides, the electrode portion 430 can have working electrodes corresponding to the trap channels 140 in number.

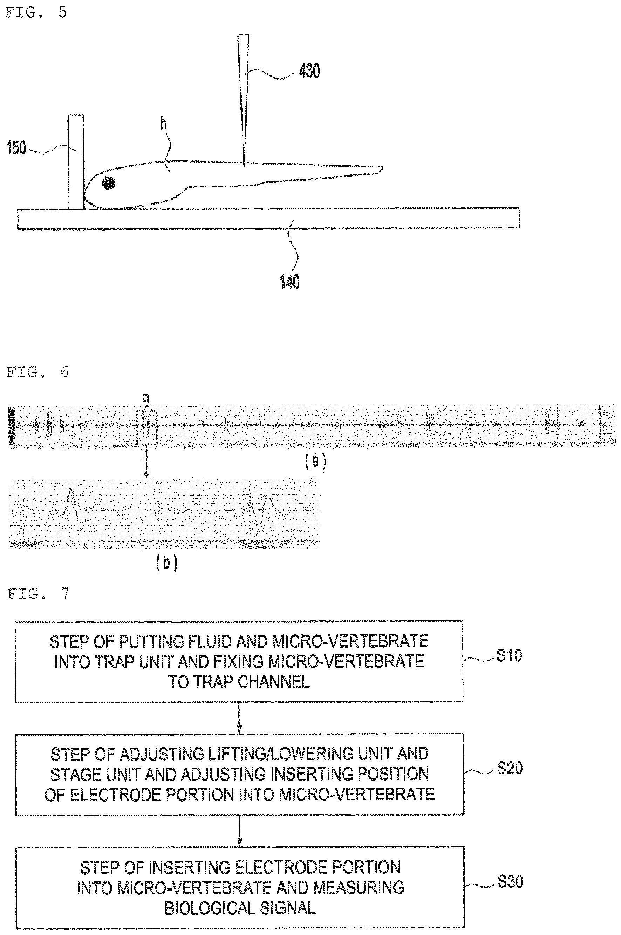

FIG. 5 is a view illustrating a state of measuring biological signals of micro-vertebrates fixed to the trap unit according to the embodiment of the present invention.

As illustrated in FIG. 5, in the lifting/lowering unit 400 provided as described above, a position of the vertical frame 410 can be adjusted such that the electrode portions 430 are positioned above micro-vertebrates fixed to the trap channels 140. Besides, the horizontal frame 420 can be lifted and lowered to cause the electrode portions 430 to be inserted into the micro-vertebrates. At this point, the electrode portion 430 can measure a potential difference between the working electrode and the comparison electrode 150 provided in the trap unit 100 so as to measure biological signals of the micro-vertebrates.

FIG. 6 is a graph showing a result of electromyogram measurement of micro-vertebrates by using the biological-signal measuring apparatus equipped with a trap unit for measuring biological signals of micro-vertebrates according to the embodiment of the present invention.

As illustrated in FIG. 6, the biological-signal measuring apparatus 1000 equipped with a trap unit for measuring biological signals of micro-vertebrates can be applied to an electromyography system, an electrocardiography system, and a brainwave test system, the biological-signal measuring apparatus being provided as described above.

Here, the electromyography, the electrocardiography, and the brainwave test can be easily performed by changing an inserting position of the electrode portion 430 into the micro-vertebrates. That is, according to the present invention, an inserting position of the electrode portion 430 can be simply adjusted, and thereby various tests such as the electromyography, the electrocardiography, and the brainwave test can be rapidly and easily performed.

FIG. 7 is a flowchart of a method for measuring biological signals of micro-vertebrates by using the biological-signal measuring apparatus equipped with a trap unit for measuring biological signals of micro-vertebrates, the method not falling within the scope of the claims.

As illustrated in FIG. 7, the method for measuring biological signals of micro-vertebrates by using the biological-signal measuring apparatus equipped with a trap unit for measuring biological signals of micro-vertebrates includes Step S10 of putting a fluid and micro-vertebrates into the trap unit and fixing the micro-vertebrates to the trap channels, Step S20 of adjusting the lifting/lowering unit and the stage unit and adjusting an inserting position of the electrode portion into the micro-vertebrates, and Step S30 of inserting the electrode portion into the micro-vertebrates and measuring biological signals.

Specifically, in Step S10 of putting the fluid and the micro-vertebrates into the trap unit and fixing the micro-vertebrates to the trap channels, the fluid and the micro-vertebrates can be first put into the injection chamber 120. Besides, the micro-vertebrates injected into the injection chamber 120 can move individually through the transfer channel 130 to the respective trap channels 140. At this point, the head part h of the micro-vertebrates can be caught and fixed to a trap having a size corresponding to that of the head part h.

In Step S20 of adjusting the lifting/lowering unit and the stage unit and adjusting the inserting position of the electrode portion into the micro-vertebrates, the stage unit 200 and the lifting/lowering unit 400 can adjust the electrode portions 430 to be positioned above the micro-vertebrates fixed to the trap channels 140. More specifically, the inserting position of the electrode portion 430 into the micro-vertebrates is different depending on testing purposes of the electromyography, the electrocardiography, the electroencephalography, or the like. Hence, the stage unit 200 and the lifting/lowering unit 400 can be adjusted with consideration for the inserting position of the electrode portion 430 into the micro-vertebrates, depending on a testing purpose.

In Step S30 of inserting the electrode portion into the micro-vertebrates and measuring biological signals, the electrode portion 430 can be inserted into the micro-vertebrates as the horizontal frame 420 is lowered. At this point, the electrode portion 430 can measure a potential difference between the working electrode and the comparison electrode 150 provided in the trap unit 100 so as to measure biological signals of the micro-vertebrates.

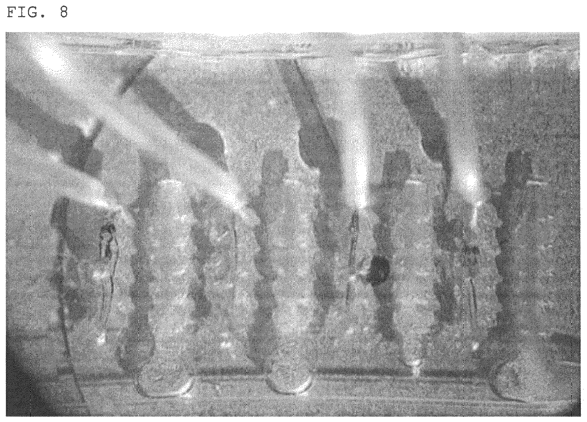

FIG. 8 is a picture showing a state of measuring the biological signals of the micro-vertebrates fixed to the trap unit according to the embodiment of the present invention.

As described above, the biological-signal measuring apparatus 1000 equipped with a trap unit for measuring biological signals of the micro-vertebrates can simply and rapidly fix a plurality of micro-vertebrates almost simultaneously without mechanical or physical control, can easily and simply adjust the inserting portions of the electrode portions 430, and thus can rapidly measure desired biological signals of the plurality of individual micro-vertebrates.

In addition, according to the present invention, it is possible to easily change a drug which is to be injected into the fixed micro-vertebrate, and thus it is possible to perform a simple experiment.

In addition, according to the present invention, since the micro-vertebrates are to be fixed in the fluid, the life of the micro-vertebrates can be maintained for a long time, and thus it is possible to obtain more measurement data from one micro-vertebrate and to measure various biological signals.

That is, the present invention enables mass measurement of biological signals of the micro-vertebrates and, thus, is economical.

The description of the present invention described above is provided as an example, and a person of ordinary skill in the art to which the present invention belongs can understand that it is possible to easily modify the present invention to another embodiment without altering the technical idea or an essential feature of the present invention. Therefore, the embodiments described above need to be understood as exemplified examples in every aspect and not as examples limiting the present invention. For example, the configurational elements described in singular forms can be realized in a distributed manner. Similarly, the configurational elements described in the distributed manner may be realized in a combined manner.

### [Reference Signs List]

- 100: TRAP UNIT
- 110: SUBSTRATE
- 120: INJECTION CHAMBER
- 130: TRANSFER CHANNEL
- 140, 190: TRAP CHANNELS
- 150: COMPARISON ELECTRODE
- 160: DISCHARGE SECTION
- 200: STAGE UNIT
- 210: INSERTION BODY
- 220: FIXING BODY
- 230: SLIDING SECTION
- 240: MAIN STAGE BODY
- 250: WIDTH ADJUSTER
- 260: LENGTH ADJUSTER
- 300: BASE UNIT
- 310: BASE SECTION
- 320: RAIL SECTION
- 400: LIFTING/LOWERING UNIT
- 410: VERTICAL FRAME
- 420: HORIZONTAL FRAME
- 430: ELECTRODE PORTION
- 1000: BIOLOGICAL-SIGNAL MEASURING APPARATUS EQUIPPED WITH
- TRAP: UNIT FOR MEASURING BIOLOGICAL SIGNAL OF MICRO-VERTEBRATE

## Claims

1. A biological-signal measuring apparatus (1000) equipped with a trap unit for measuring biological signals of micro-vertebrates, wherein the micro-vertebrates are zebrafish, the biological-signal measuring apparatus (1000) comprising:
a trap unit (100) having multiple trap channels (140) to which micro-vertebrates are fixable; and
wherein the trap channels (140) have a shape formed to fix the head part (h) of the micro-vertebrates put into the trap unit (100),
wherein the trap unit (100) has
an injection chamber (120) into which the micro-vertebrates and a fluid are injectable;
a transfer channel (130) that is provided to be extended from the injection chamber (120) toward one side and forms a flow channel to transfer the micro-vertebrates and the fluid injected into the injection chamber (120) to the trap channels (140); and
one or more trap channels (140) which are provided to be extended from the transfer channel in one direction; **characterized in that** the apparatus (1000) further comprises
a lifting/lowering unit (400) configured to insert an electrode portion (430) into the micro-vertebrates fixed to the trap unit (100) and measure biological signals,
a discharge section (160),
wherein the trap channels(140, 190) each have a plurality of traps formed in a length direction of the trap channels(140, 190), and
wherein the traps have a sectional area which decreases as being positioned downstream such that a trap (141b) positioned downstream has a smaller sectional area than a trap (141a) positioned relatively upstream.

2. The biological-signal measuring apparatus (1000) equipped with a trap unit for measuring biological signals of micro-vertebrates according to claim 1,
wherein the trap unit (100) further has comparison electrodes (150) provided to be in contact with the micro-vertebrates when each of the micro-vertebrates are fixed to the trap channels (140), and
wherein, when the electrode portion (430) is inserted into the micro-vertebrates, a potential difference between the electrode portion (430) and the comparison electrode (150) is measured to measure biological signals of the micro-vertebrates.

3. The biological-signal measuring apparatus (1000) equipped with a trap unit for measuring biological signals of micro-vertebrates according to claim 1, further comprising:
a stage unit (200) that is provided below the trap unit (100) and configured to control a position of the trap unit (100); and
a base unit (300) to which, on an upper side, the stage unit (200) and the lifting/lowering unit (400) are coupled.

4. The biological-signal measuring apparatus (1000) equipped with a trap unit for measuring biological signals of micro-vertebrates according to claim 3,
wherein the stage unit (200) has
an insertion body (210) having inside a groove into which the trap unit (100) is inserted and fixed;
a fixing body (220) which is provided on an outer surface of the insertion body (210) and configured to fix the insertion body (210);
a sliding section (230) which is provided below the insertion body (210) and is provided to be slidable in an X direction and a Y direction;
a main stage body (240) which is provided below the sliding section (230);
a width adjuster (250) which is connected to the main stage body (240) and configured to move the sliding section (230) in the X direction; and
a length adjuster (260) which is connected to the main stage body (240) and configured to move the sliding section (230) in the Y direction.

5. The biological-signal measuring apparatus (1000) equipped with a trap unit for measuring biological signals of micro-vertebrates according to claim 3,
wherein the base unit (300) has
a base section (310) which is coupled to the lifting/lowering unit (400) and the stage unit (200) at a top part of the base section (310) and forms a body; and
a rail section (320) on which the lifting/lowering unit (400) is slidable in a length direction of the base section (310).

6. The biological-signal measuring apparatus (1000) equipped with a trap unit for measuring biological signals of micro-vertebrates according to claim 6,
wherein the lifting/lowering unit (400) further has
a vertical frame (410) which is configured to slide along the rail section (320) in the length direction of the base section (310);
a horizontal frame (420) provided to be extended from the vertical frame (410) in one direction so as to be liftable and lowerable in a length direction of the vertical frame (410); and
the electrode portion (430) which is coupled to the horizontal frame (420) and is extended downward, and
wherein the electrode portion (430) includes working electrodes corresponding to the trap channels (140) in number.

7. The biological-signal measuring apparatus (1000) according to any one of claims 1-6 and an
electromyography system using the biological-signal measuring apparatus (1000).

8. The biological-signal measuring apparatus (1000) according to any one of claims 1-6 and an electrocardiography system using the biological-signal measuring apparatus (1000).

9. The biological-signal measuring apparatus (1000) according to any one of claims 1-6 and an electroencephalography or a brainwave test system using the biological-signal measuring apparatus (1000).

## Patentansprüche

1. Eine Messvorrichtung (1000) für biologische Signale, die mit einer Einfangeinheit ausgerüstet ist, zum Messen biologischer Signale von Mikro-Wirbeltieren, wobei die Mikro-Wirbeltiere Zebrafische sind, wobei die Messvorrichtung (1000) für biologische Signale folgende Merkmale aufweist:
eine Einfangeinheit (100) mit mehreren Einfangkanälen (140), an denen Mikro-Wirbeltiere fixierbar sind; und
wobei die Einfangkanäle (140) eine Form aufweisen, die so gebildet ist, dass sie den Kopfteil (h) der Mikro-Wirbeltiere fixiert, die in die Einfangeinheit (100) gegeben werden,
wobei die Einfangeinheit (100) folgende Merkmale aufweist:
eine Einspritzkammer (120), in die die Mikro-Wirbeltiere und ein Fluid einspritzbar sind;
einen Übertragungskanal (130), der so vorgesehen ist, dass er sich von der Einspritzkammer (120) in Richtung einer Seite erstreckt, und einen Strömungskanal bildet, um die Mikro-Wirbeltiere und das Fluid, die in die Einspritzkammer (120) eingespritzt sind, zu den Einfangkanälen (140) zu übertragen; und
einen oder mehr Einfangkanäle (140), die so vorgesehen sind, dass sie sich von dem Übertragungskanal in einer Richtung erstrecken;
**dadurch gekennzeichnet, dass** die Vorrichtung (1000) ferner folgende Merkmale aufweist:
eine Anhebe-/Absenkeinheit (400), die dazu ausgebildet ist, einen Elektrodenabschnitt (430) in die Mikro-Wirbeltiere einzuführen, die an der Einfangeinheit (100) fixiert sind, und biologische Signale zu messen,
einen Abgabeabschnitt (160),
wobei die Einfangkanäle (140, 190) jeweils eine Mehrzahl von Fallen aufweisen, die in einer Längenrichtung der Einfangkanäle (140, 190) gebildet sind, und
wobei die Fallen eine Querschnittsfläche aufweisen, die mit einer in Verarbeitungsrichtung nachgelagerten Positionierung abnimmt, so dass eine Falle (141b), die in Verarbeitungsrichtung nachgelagert positioniert ist, eine kleinere Querschnittsfläche aufweist als eine Falle (141a), die relativ in Verarbeitungsrichtung vorgelagert positioniert ist.

2. Die Messvorrichtung (1000) für biologische Signale, die mit einer Einfangeinheit ausgerüstet ist, zum Messen biologischer Signale von Mikro-Wirbeltieren gemäß Anspruch 1,
bei der die Einfangeinheit (100) ferner Vergleichselektroden (150) aufweist, die so vorgesehen sind, dass sie in Kontakt mit den Mikro-Wirbeltieren stehen, wenn jedes der Mikro-Wirbeltiere an den Einfangkanälen (140) fixiert ist, und
wobei, wenn der Elektrodenabschnitt (430) in die Mikro-Wirbeltiere eingeführt ist, ein Potenzialunterschied zwischen dem Elektrodenabschnitt (430) und der Vergleichselektrode (150) gemessen wird, um biologische Signale der Mikro-Wirbeltiere zu messen.

3. Die Messvorrichtung (1000) für biologische Signale, die mit einer Einfangeinheit ausgerüstet ist, zum Messen biologischer Signale von Mikro-Wirbeltieren gemäß Anspruch 1, die ferner folgende Merkmale aufweist:
eine Stufeneinheit (200), die unterhalb der Einfangeinheit (100) vorgesehen und dazu ausgebildet ist, eine Position der Einfangeinheit (100) zu steuern; und
eine Basiseinheit (300), mit der an einer Oberseite die Stufeneinheit (200) und die Anhebe-/Absenkeinheit (400) gekoppelt sind.

4. Die Messvorrichtung (1000) für biologische Signale, die mit einer Einfangeinheit ausgerüstet ist, zum Messen biologischer Signale von Mikro-Wirbeltieren gemäß Anspruch 3,
bei der die Stufeneinheit (200) folgende Merkmale aufweist:
einen Einführkörper (210), der im Inneren eine Rille aufweist, in die die Einfangeinheit (100) eingeführt und fixiert ist;
einen Fixierkörper (220), der an einer Außenoberfläche des Einführkörpers (210) vorgesehen und dazu ausgebildet ist, den Einführkörper (210) zu fixieren;
einen Gleitabschnitt (230), der unterhalb des Einführkörpers (210) vorgesehen ist und so vorgesehen ist, dass er in einer X-Richtung und einer Y-Richtung gleitfähig ist;
einen Hauptstufenkörper (240), der unterhalb des Gleitabschnitts (230) vorgesehen ist;
einen Breitenanpasser (250), der mit dem Hauptstufenkörper (240) verbunden und dazu ausgebildet ist, den Gleitabschnitt (230) in der X-Richtung zu bewegen; und
einen Längenanpasser (260), der mit dem Hauptstufenkörper (240) verbunden und dazu ausgebildet ist, den Gleitabschnitt (230) in der Y-Richtung zu bewegen.

5. Die Messvorrichtung (1000) für biologische Signale, die mit einer Einfangeinheit ausgerüstet ist, zum Messen biologischer Signale von Mikro-Wirbeltieren gemäß Anspruch 3,
bei der die Basiseinheit (300) folgende Merkmale aufweist:
einen Basisabschnitt (310), der mit der Anhebe-/Absenkeinheit (400) und der Stufeneinheit (200) an einem oberen Teil des Basisabschnitts (310) gekoppelt ist und einen Körper bildet; und
einen Schienenabschnitt (320), an dem die Anhebe-/Absenkeinheit (400) in einer Längenrichtung des Basisabschnitts (310) gleitfähig ist.

6. Die Messvorrichtung (1000) für biologische Signale, die mit einer Einfangeinheit ausgerüstet ist, zum Messen biologischer Signale von Mikro-Wirbeltieren gemäß Anspruch 6,
bei der die Anhebe-/Absenkeinheit (400) ferner folgende Merkmale aufweist:
einen Vertikalrahmen (410), der dazu ausgebildet ist, in der Längenrichtung des Basisabschnitts (310) entlang des Schienenabschnitts (320) zu gleiten;
einen Horizontalrahmen (420), der so vorgesehen ist, dass er sich von dem Vertikalrahmen (410) in einer Richtung erstreckt, um so in einer Längenrichtung des Vertikalrahmens (410) anhebbar und absenkbar zu sein; und
den Elektrodenabschnitt (430), der mit dem Horizontalrahmen (420) gekoppelt ist und sich nach unten erstreckt, und
wobei der Elektrodenabschnitt (430) Arbeitselektroden umfasst, die zahlenmäßig den Einfangkanälen (140) entsprechen.

7. Die Messvorrichtung (1000) für biologische Signale gemäß einem der Ansprüche 1-6 und ein Elektromyographiesystem, das die Messvorrichtung (1000) für biologische Signale verwendet.

8. Die Messvorrichtung (1000) für biologische Signale gemäß einem der Ansprüche 1-6 und ein Elektrokardiographiesystem, das die Messvorrichtung (1000) für biologische Signale verwendet.

9. Die Messvorrichtung (1000) für biologische Signale gemäß einem der Ansprüche 1-6 und ein Elektroenzephalographie- oder Hirnwellentestsystem, das die Messvorrichtung (1000) für biologische Signale verwendet.

## Revendications

1. Appareil de mesure de signaux biologiques (1000) équipé d'une unité de piège destinée à mesurer les signaux biologiques de micro-vertébrés, dans lequel les micro-vertébrés sont des poissons zèbres, l'appareil de mesure de signaux biologiques (1000) comprenant:
une unité de piège (100) présentant de multiples canaux de piège (140) auxquels peuvent être fixés les micro-vertébrés; et
dans lequel les canaux de piège (140) présentent une forme formée pour fixer la partie de tête (h) des micro-vertébrés introduits dans l'unité de piège (100),
dans lequel l'unité de piège (100) présente
une chambre d'injection (120) dans laquelle peuvent être injectés les micro-vertébrés et un fluide;
un canal de transfert (130) qui est prévu pour s'étendre à partir de la chambre d'injection (120) vers un côté et qui forme un canal de circulation pour transférer les micro-vertébrés et le fluide injecté dans la chambre d'injection (120) vers les canaux de piège (140); et
un ou plusieurs canaux de piège (140) qui sont prévus pour s'étendre à partir du canal de transfert dans une direction; **caractérisé par le fait que** l'appareil (1000) comprend par ailleurs
une unité de levage/d'abaissement (400) configurée pour insérer une partie d'électrode (430) dans les micro-vertébrés fixés à l'unité de piège (100) et pour mesurer les signaux biologiques,
un segment de décharge (160),
dans lequel les canaux de piège (140, 190) présentent, chacun, une pluralité de pièges formés dans la direction de la longueur des canaux de piège (140, 190), et
dans lequel les pièges présentent une surface de section qui diminue au fur et à mesure qu'ils sont positionnés en aval, de sorte qu'un piège (141b) positionné en aval présente une surface de section plus petite qu'un piège (141a) positionné relativement en amont.

2. Appareil de mesure de signaux biologiques (1000) équipé d'une unité de piège destinée à mesurer les signaux biologiques de micro-vertébrés selon la revendication 1,
dans lequel l'unité de piège (100) présente par ailleurs des électrodes de comparaison (150) prévues pour être en contact avec les micro-vertébrés lorsque chacun des micro-vertébrés est fixé aux canaux de piège (140), et
dans lequel, lorsque la partie d'électrode (430) est insérée dans les micro-vertébrés, une différence de potentiel entre la partie d'électrode (430) et l'électrode de comparaison (150) est mesurée pour mesurer les signaux biologiques des micro-vertébrés.

3. Appareil de mesure de signaux biologiques (1000) équipé d'une unité de piège destinée à mesurer les signaux biologiques de micro-vertébrés selon la revendication 1, comprenant par ailleurs:
une unité d'étage (200) qui est prévue au-dessous de l'unité de piège (100) et qui est configurée pour commander une position de l'unité de piège (100); et
une unité de base (300) à laquelle sont couplées, sur un côté supérieur, l'unité d'étage (200) et l'unité de levage/d'abaissement (400).

4. Appareil de mesure de signaux biologiques (1000) équipé d'une unité de piège destinée à mesurer les signaux biologiques de micro-vertébrés selon la revendication 3,
dans lequel l'unité d'étage (200) présente
un corps d'insertion (210) présentant à l'intérieur une rainure dans laquelle est insérée et fixée l'unité de piège (100);
un corps de fixation (220) qui est prévu sur une surface extérieure du corps d'insertion (210) et qui est configuré pour fixer le corps d'insertion (210);
un segment coulissant (230) qui est prévu au-dessous du corps d'insertion (210) et qui est prévu pour pouvoir coulisser dans une direction X et une direction Y;
un corps d'étage principal (240) qui est prévu au-dessous du segment coulissant (230);
un moyen de réglage de largeur (250) qui est connecté au corps d'étage principal (240) et qui est configuré pour déplacer le segment coulissant (230) dans la direction X; et
un moyen de réglage de longueur (260) qui est connecté au corps d'étage principal (240) et qui est configuré pour déplacer le segment coulissant (230) dans la direction Y.

5. Appareil de mesure de signaux biologiques (1000) équipé d'une unité de piège destinée à mesurer les signaux biologiques de micro-vertébrés selon la revendication 3,
dans lequel l'unité de base (300) présente
un segment de base (310) qui est couplé à l'unité de levage/d'abaissement (400) et à l'unité d'étage (200) au niveau d'une partie supérieure du segment de base (310) et qui forme un corps; et
un segment de rail (320) sur lequel l'unité de levage/d'abaissement (400) peut coulisser dans la direction de la longueur du segment de base (310).

6. Appareil de mesure de signaux biologiques (1000) équipé d'une unité de piège destinée à mesurer les signaux biologiques de micro-vertébrés selon la revendication 6,
dans lequel l'unité de levage/d'abaissement (400) présente par ailleurs
un cadre vertical (410) qui est configuré pour glisser le long du segment de rail (320) dans la direction de la longueur du segment de base (310);
un cadre horizontal (420) prévu pour s'étendre à partir du cadre vertical (410) dans une direction de manière à pouvoir être levé et abaissé dans une direction longitudinale du cadre vertical (410); et
la partie d'électrode (430) qui est couplée au cadre horizontal (420) et qui s'étend vers le bas, et
dans lequel la partie d'électrode (430) comporte des électrodes de travail correspondant aux canaux de piège (140) en nombre.

7. Appareil de mesure de signaux biologiques (1000) selon l'une quelconque des revendications 1 à 6 et système d'électromyographie utilisant l'appareil de mesure de signaux biologiques (1000).

8. Appareil de mesure de signaux biologiques (1000) selon l'une quelconque des revendications 1 à 6 et système d'électrocardiographie utilisant l'appareil de mesure de signaux biologiques (1000).

9. Appareil de mesure de signaux biologiques (1000) selon l'une quelconque des revendications 1 à 6 et système d'électroencéphalographie ou de test des ondes cérébrales utilisant l'appareil de mesure de signaux biologiques (1000).
